# EUROPÄISCHE PATENTSCHRIFT

(11) **EP 0 466 681 B1**
(45) Veröffentlichungstag und Bekanntmachung des Hinweises auf die Patenterteilung: **08.11.1995**
(21) Anmeldenummer: 91890142.2
(22) Anmeldetag: 05.07.1991
(51) Int. Cl.: A61N 5/10

(54) **Strahlerhalter sowie Verfahren und Einrichtung zur Herstellung desselben**
Seedholder and process and device for its manufacture
Magasin à sources radioactives, et procédé et dispositif pour sa fabrication

(30) Priorität: 11.07.1990 AT 1471/90
(43) Veröffentlichungstag der Anmeldung: 15.01.1992
(73) Patentinhaber: Österreichisches Forschungszentrum Seibersdorf Ges.m.b.H., 1010 Wien (AT)
(72) Erfinder: Görz, Walter, A-2444 Seibersdorf (AT); Nedelik, Adolf, A-1100 Wien (AT); Miksche, Wolfgang, A-23333 Leopoldsdorf (AT); Bendl, Dietfried, A-1100 Wien (AT)

(56) Entgegenhaltungen:
- DE-B- 1 065 989
- DE-B- 1 095 963
- US-A- 3 964 468
- British Journal of Radiology vol. 48, no. 568, April 1975, London GB Seiten 295- 298; Haybittle et al: "A simple after-loading technique for the treatment ofcancer of the cervix"

## Beschreibung

Die Erfindung betrifft einen Strahlerhalter zum Einsatz in interstitiell eingebrachte Bestrahlungsnadeln, umfassend eine Kunststoffhülle und zumindest ein in dieser angeordnetes, vorzugsweise langgestrecktes, radioaktives Seed. Ferner betrifft die Erfindung ein Verfahren zur Herstellung radioaktiver Strahlerhalter zum Einsatz in interstitiell eingebrachte Bestrahlungsnadeln und eine Einrichtung zur Herstellung derartiger Strahlerhalter zum Einsatz in interstitiell eingebrachte Bestrahlungsnadeln.

Bekannt ist es, daß ein Strahlerhalter mittels einer Pinzette von Hand aus oder mit einer Handhabungsvorrichtung gemäß der AT-PS 385 195 in Hohlnadeln zur interstitiellen Bestrahlung eingebracht wird. Dabei ergibt sich ein erhöhter Aufwand zur Fixierung des aus einem radioaktiven Drahtstück bestehenden Seeds und der Spitze des Strahlerhalters. Aufgrund der bei der Manipulation entstehenden hohen Strahlenbelastung bringt diese Anwendung Strahlenschutzprobleme mit sich. Ferner ist es bekannt, aktive Strahlenquellen in Kunststoffumhüllungen lose einzuführen, mit der Gefahr, daß die Strahlenquellen ihre Position verändern können bzw. im Falle eines Bruches der Umhüllung die Strahlenquellen austreten können.

Aus der DE-A1-1,065.989 ist weiters ein Strahlerhalter zum Einsatz in interstitiell eingebrachte Bestrahlungsnadeln, umfassend eine Kunststoffhülle und zumindest ein in diese angeordnetes radioaktives Seed bekannt, wobei die Strahlungsquelle(n) in einer zylindrischen Hülse aus Gummi oder Kunststoff eingelegt ist. Beispielsweise ist die Strahlungsquelle mit einer Mehrzahl von, durch kurze Abstände getrennten, Drahtstückchen aus radioaktivem Material gebildet. Die Lagehaltung der Seeds erfolgt dort durch reversible, lokale Radial-Dehnung der elastischen Hülse an den Stellen, wo sich die Seeds befinden. Die Abschnitte zwischen den Strahlerelementen weisen weiterhin Elastizität auf, somit aber die Lage-Haltung der Seeds relativ unsicher bleibt und bei Einwirkung relativ geringer Kräfte, z.B. bei Manipulation oder beim Einführen, unerwünschte Relativverschiebungen der Seeds erfolgen können.

Ergänzend sei zum Stand der Technik noch auf die DE-A1-1,095.963 verwiesen, welche eine Einrichtung zur Beschickung eines Applikators mit radioaktiven Strahlerelementen zum Gegenstand hat, sowie auf die Literaturstelle J.L.Haybittle et al. "A simple after-loading technique for the treatment of cancer of the cervix" in British J.Radiology, April 1975, pp.295-298, aus welcher die Anordnung von länglichen radioaktiven Seeds in röhrenartige PVC-Nadeln ohne eigene Hülle hervorgeht.

Ziel der Erfindung ist es, diese vorbeschriebenen Nachteile zu beheben und bei hoher Biegsamkeit des Strahlerhalters zur Optimierung der Tumorbestrahlungs-Geometrie eine gegen äußere Einwirkung unempfindliche echte Lagefixierung der Seeds zu erreichen. Weiteres Ziel der Erfindung ist es, eine hohe Flexibilität bei der Einführung von Low-Dose-radioaktiven Elementen bzw. Seeds in den menschlichen Körper bzw. in Bestrahlungsnadeln zu erreichen, bei gleichzeitig genauer Dosierbarkeit und Positionierbarkeit.

Erfindungsgemäß ist ein Strahlerhalter der eingangs genannten Art dadurch gekennzeichnet, daß die Kunststoffhülle von einer bzw. einem auf das zumindest eine Seed unter dessen Lagefixierung thermisch aufgeschrumpften Kunststoffumhüllung bzw. Kunststoffschrumpfschlauch gebildet ist. Durch das Einbringen kurzer Drahtstücke in den Kunststoffschrumpfschlauch und nachträgliches Aufschrumpfen wird die Beweglichkeit des Seeds in der Bestrahlungsnadel verbessert. Gleichzeitig kann der Schrumpfschlauch des Strahlerhalters zur Fixierung der Spitze und/oder einem Kupplungsteil zum Ankuppeln an eine Handhabungsvorrichtung, z.B. einen Drahtschieber, dienen. Der Einsatz eines Schrumpfschlauches ermöglicht eine sichere Befestigung des vorderen und hinteren Endabschlusses des Strahlerhalters, da die auf die Endteile aufgeschrumpften Kunststoff-Schrumpfschläuche ausgesprochen fest mit diesen verbunden werden können. Ferner wird eine Fixierung der Seeds in der Umhüllung erreicht, welche Tatsache ausgesprochen wichtig für die Dosisberechnung ist. Bedingt durch die erfindungsgemäße Messung der Aktivität der einzelnen Seeds nach der Neutronenaktivierung im Reaktor und der Sortierung, vorzugsweise gemäß ± 3% Aktivitätsschwankung ergeben sich qualitativ weitaus höherstehendere Strahlerhalter, als es bei den bisherigen Ausführungsformen erreichbar war; dies zeigte sich auch in praktischen Versuchen, in denen die Lage und die Aktivität der Seeds und damit die an den menschlichen Körper abgegebene Dosis weitaus besser bestimmbar war als bisher. Der erfindungsgemäße Strahlerhalter vereinbart somit den Vorteil eines leicht befüllbaren Schlauches mit einer Lagefixierung der eingefüllten Seeds; als weiterer Vorteil ergibt es sich, daß im Falle eines Bruches einer Umhüllung die im Kunststoffschrumpfschlauch befindlichen Seeds lagefest gehalten werden und durch die Bruchstelle nicht austreten können. Für die Qualität der Therapie und für die Sicherheit des Hantierenden bzw. Behandelten werden somit wesentliche Vorteile erreicht.

Der Strahlerhalter kann eine Anzahl von hintereinander in den Schrumpfschlauch eingeschrumpften gegebenenfalls bezüglich des radioaktiven Materials unterschiedliche Seeds umfassen. Auf diese Weise können Strahlerhalter vorgegebener Dosis erstellt werden, die entsprechend den verschiedenen Anwendungszwecken in die Bestrahlungsnadeln eingebracht werden können; die Reihenfolge und Lage der Seeds in der Bestrahlungsnadel wird beim Befüllen vorgegeben und bleibt danach unverändert. Zur genaueren Einregelung der Strahlungsdosis der von dem Strahlerhalter abgegebenen Strahlung kann vorgesehen sein, daß der Schrumpfschlauch in vorgegebener Aufeinanderfolge radioaktive Seeds und nicht radioaktive Füllteile bzw. Distanzstücke umfaßt. Die Seeds und die Füllteile können abwechselnd oder in bestimmter Aufeinanderfolge in den Schrumpfschlauch eingeschrumpft werden, sodaß eine Strahlenquelle mit über ihrer Länge definierter Strahlung erstellt wird.

Vorteilhafterweise wird ein transparenter Schrumpfschlauch eingesetzt, der eine Schrumpftemperatur von etwa 100°C und einen Schrumpfgrad in radialer Richtung von etwa 50 % besitzt. Die Transparenz des Schrumpfschlauches ist für die optische Feststellung seines Inhalts maßgeblich; ferner kann die Befüllung des Schrumpfschlauches mittels optischer Detektoren bzw. Lichtschranken bzw. eines Diodenmeßsystems überwacht werden. Eine Schrumpfungstemperatur von etwa 100°C ist zweckmäßig, um nicht aufwendige Schrumpfungseinrichtungen zur Verfügung stellen zu müssen; der gewählte Schrumpfungsgrad in radialer Richtung ergibt einen gut biegbaren langgestreckten Körper des Strahlerhalters, der in gerade oder gekrümmte Bestrahlungsnadeln bzw. -schläuchen gut ein- und ausfahrbar ist.

Ein Verfahren der eingangs genannten Art ist erfindungsgemäß dadurch gekennzeichnet, daß zumindest ein radioaktives Seed bzw. eine Anzahl von radioaktiven Seeds in einen, vorzugsweise transparenten und gegebenenfalls bereits abgelängten Schrumpfschlauch eingeführt werden, und daß daraufhin der Schlauch geschrumpft wird. Vorteilhaft ist es dabei, wenn zusätzlich zu den Seeds eine Anzahl von nicht radioaktiven Füllteilen bzw. Distanzstücken in vorgegebener Aufeinanderfolge mit den Seeds in den Schrumpfschlauch eingebracht wird.

Bei einer bevorzugten Ausführungsform der Erfindung ist vorgesehen, daß vor dem Einbringen der Seeds bzw. der Füllteile eine Seele, z.B. ein Stahldraht, in den Schrumpfschlauch eingeführt wird, deren Durchmesser größer als der Durchmesser der Seeds und Füllstücke ist, und daß der Schrumpfschlauch auf den Durchmesser der Seele vorgeschrumpft wird. Bei dieser Vorgangsweise liegt zum Befüllen ein Schrumpfschlauch definierten und ziemlich genau runden Durchmessers vor, der leicht befüllt werden kann.

Eine Einrichtung der eingangs genannten Art ist erfindungsgemäß dadurch gekennzeichnet, daß eine Führungshülse vorgesehen ist, an bzw. in die ein Schrumpfschlauch ansetzbar bzw. einsteckbar ist, und daß die Führungshülse zur Einführung der Seeds in den Schrumpfschlauch an das Ende einer von einer Rütteleinrichtung für radioaktive Seeds kommenden, vorzugsweise durchsichtigen Transportleitung anlegbar bzw. ankoppelbar ist und daß der Einrichtung eine Heizeinrichtung, z.B. ein Heißlufterzeuger, zum Schrumpfen des Schrumpfschlauches nachgeordnet ist, durch die der gefüllte Schlauch mit einer Transporteinrichtung durchbewegbar ist.

Eine bevorzugte Ausführungsform der erfindungsgemäßen Einrichtung sieht vor, daß die Führungsteile mittels einer Umschalteinrichtung bzw. einer Weiche in willkürlicher Weise an die Transportleitung der Rütteleinrichtungen für die Seeds und eine Transportleitung einer Rütteleinrichtung für nichtaktive Füllteile bzw. Distanzstücke anschließbar ist.

Im folgenden wird die Erfindung beispielsweise anhand der Zeichnung näher erläutert. Es zeigen Fig.1 eine schematische Ansicht einer Vorrichtung zum Befüllen eines Schlauches, Fig.1a eine Detailansicht und Fig.2 und 3 Herstellungsschritte bei der Herstellung eines erfindungsgemäßen Strahlerhalters.

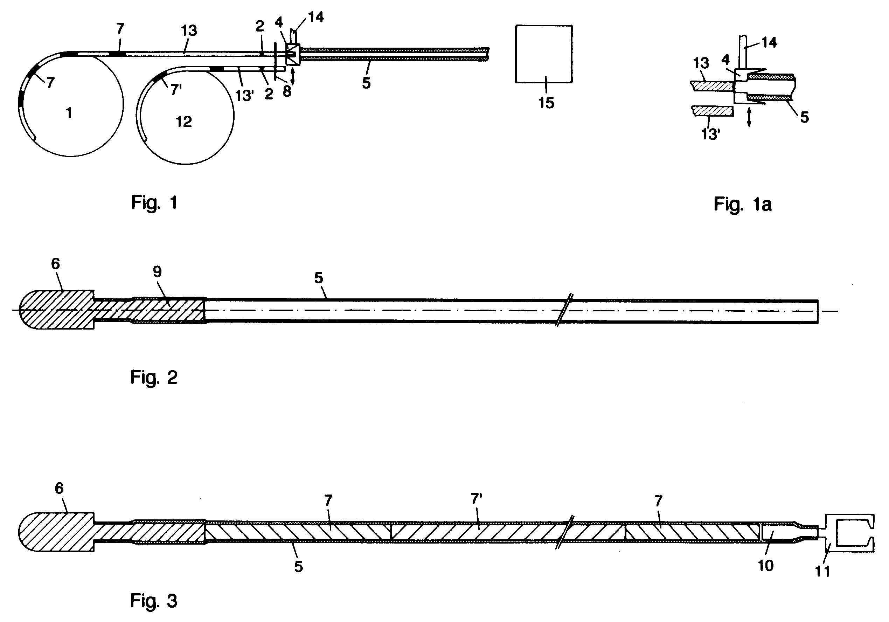

In Fig.1 ist mit 1 schematisch eine Rüttelvorrichtung für radioaktive Seeds und mit 12 eine Rüttelvorrichtung für nicht radioaktive Füllteile dargestellt, von welchen beiden Einrichtungen 1 und 12 jeweils ein Transportschlauch 13 bzw. 13' abgeht, die mit Seeds 7 bzw. Füllteilen 7' beschickt werden. Das Ende jedes Transportschlauches 13,13' ist mit einer Führungshülse 4 koppelbar, die einen Kunststoffschlauch bzw. Schrumpfschlauch 5 führt, wie Fig.1a zeigt. Die Hülse 4 ist auf einem Träger 14, der von einer Antriebseinrichtung betätigt ist, zwischen Stellungen verstellbar, in denen ihre Bohrung mit den Transportschläuchen 13 bzw. 13' fluchtet. Der Schrumpfschlauch 5 ist in die Hülse 4 eingesteckt und leicht lösbar. In diesen Kunststoffschlauch 5 kann bereits eine Spitze 6 an dem der Stütze 4 abgewandten Ende angebracht bzw. eingesetzt sein, so wie dies in Fig.2, in größerem Maßstab dargestellt ist.

Das Befüllen des Schrumpfschlauches 5 erfolgt durch Einrütteln der Seeds 7 in den Schlauch oder durch Einschieben mit Hilfe eines Drahtstößels od.dgl. Wenn der Schlauch 5 ausreichend gefüllt ist, wird die Zufuhr der Seeds bzw. Füllelemente gestoppt. Die Füllkontrolle bzw. das Stoppen kann mit Hilfe einer Lichtschranke 8 erfolgen, die in den Transportschläuchen 13,13' Sperrvorrichtungen 2 betätigt, sodaß die Seeds 7 bzw. Füllteile 7' nicht mehr in den Schlauch 5 eintreten können bzw. im Transportschlauch 13,13' angehalten werden. Auf diese Weise kann ein automatisches Befüllen des Schlauches 5 unter Rechnersteuerung vorgesehen werden.

Es ist möglich, den Schrumpfschlauch 5 bereits vor seiner Befüllung abzulängen oder auch erst nachdem die Seeds 7 und Füllstücke 7' eingebracht worden sind. Die Transparenz des Schlauches ist zweckmäßig im Hinblick auf die Lichtschranke 8 und zur Feststellung des Inhaltes des Strahlerhalters. Übliche Durchmesser des Seeds liegen bei 0,3 bis 0,5 mm, insbesondere bei 0,3 bis 0,4 mm, und es werden bevorzugt Schrumpfschläuche eingesetzt, die einen Innendurchmesser von 0,6 mm besitzen und auf die Größe von 0,3 mm schrumpfbar sind.

Wie in Fig.2 dargestellt, kann in das Ende eines Schrumpfschlauches ein Endteil 9 einer Spitze 6 eingesetzt werden, wobei - wie Fig.3 zeigt - der Schrumpfschlauch 5 auf diesen Endteil 9 der Spitze 6 aufgeschrumpft werden kann. In gleicher Weise kann in das andere Ende des Schrumpfschlauches 5 eine Kupplungseinrichtung 11 mit einem Führungsteil 10 eingesetzt werden, auf welchen Führungsteil 10 das Ende des Schrumpfschlauches aufgeschrumpft wird, sodaß sich, wie in Fig.3 gezeigt, ein biegsamer, mit einer Spitze 6 und einem Kupplungsteil 11 versehener Strahlerhalter ergibt.

Vorteilhafterweise wird in die üblicherweise in unregelmäßiger Form vorliegenden Schrumpfschläuche vor dem Befüllen eine Drahtseele eingeführt, z.B. ein Stahldraht, auf den der Schlauch vorgeschrumpft wird, um seine notwendige Rundheit zu erzielen. Dieses Vorschrumpfen erfolgt insbesondere auf einem Durchmesser von 5 mm.

Daraufhin wird der mit der Drahtseele versehene vorgeschrumpfte Schlauch 5 in die Führungshülse 4 eingeführt und der Innendraht entfernt.

Darauffolgend wird der Schrumpfschlauch mittels der zur Auflockerung der Seeds 7 bzw. der Distanzstücke 7' dienenden Vorrichtung 1 und der Vorrichtung 12, die von Rüttlern, Vibratoren od.dgl. gebildet sind, gefüllt, wobei die Seeds 7 und die Füllteile 7'' in gewählter Reihenfolge in den Transportschlauch eingebracht werden.

Schließlich erfolgt das Aufschrumpfen des Kupplungsteiles 11, der mit der Spitze eines Führungs- bzw. Einführdrahtes kuppelbar ist, mit dem der Strahlerhalter in die Bestrahlungsnadel eingeführt und allenfalls daraus wieder entfernt wird.

Der befüllte und mit der Spitze 6 und Kupplungsteil 11 versehene Schlauch 5 wird daraufhin in ein Heizgerät 14, z.B. in einen Heißluftstrom, eingebracht, in dem bei etwa 100°C das Endschrumpfen stattfindet.

Die Transparenz des Schrumpfschlauches ermöglicht ein Erkennen der Seeds 7 bzw. Füllteile 7'. Ein angebrachter Code am Seed, z.B. Farbcode, Barcode od.dgl., dient zu deren Identifizierung; auch die Anbringung eines magnetischen (Bar)codes, z.B. an NiFeCo-Metallen ist möglich. Dieser vom Bestrahlungsgerät lesbare Code sichert die verwechslungsfreie Anordnung in der Klinik.

Die Form der Spitze 6 und des Kupplungsteiles 11 kann beliebig gewählt werden; ebenso ist der Durchmesser der Seeds wählbar; an den Durchmesser der Seeds wird der Innendurchmesser des Kunststoffschlauches angepaßt, wobei dessen Schrumpfverhalten zu beachten ist.

Die Schläuche können mit radioaktiven Seeds und Füllteilen aus verschiedenen Metallen bzw. Materialien gefüllt werden.

Zweckmäßig kann es sein, wenn die Aktivität der Seeds insbesondere unmittelbar vor dem Einbringen in den Schrumpfschlauch gemessen wird und nur Seeds mit vorbestimmten Aktivitätswerten (z.B. ± 3% vom vorgegebenen (Wert) in den Schrumpfschlauch eingeführt werden. Damit kann der Aktivitätsverteilung im Schrumpfschlauch ein bestimmter, gegebenenfalls konstanter, Verlauf verliehen werden.

## Patentansprüche

1. Strahlerhalter zum Einsatz in interstitiell eingebrachte Bestrahlungsnadeln, umfassend eine Kunststoffhülle (5) und zumindest ein in dieser angeordnetes, vorzugsweise langgestrecktes, radioaktives Seed (7) , dadurch gekennzeichnet, daß die Kunststoffhülle von einer bzw. einem auf das zumindest eine Seed (7) unter dessen Lagefixierung thermisch aufgeschrumpften Kunststoffumhüllung bzw. Kunststoffschrumpfschlauch (5) gebildet ist.

2. Strahlerhalter nach Anspruch 1, dadurch gekennzeichnet, daß in dem Schrumpfschlauch (5) hintereinander eine Anzahl von eingeschrumpften Seeds (7) angeordnet sind.

3. Strahlerhalter nach Anspruch 1 oder 2, dadurch gekennzeichnet, daß in dem Schrumpfschlauch (5) in vorgegebener Aufeinanderfolge radioaktive Seeds (7) und nicht radioaktive, vorzugsweise gleichen Durchmesser wie die Seeds (7) aufweisende Füllteile bzw. Distanzstücke (7') angeordnet sind.

4. Strahlerhalter nach einem der Ansprüche 1 bis 3, dadurch gekennzeichnet, daß der vorzugsweise transparente Schrumpfschlauch (5) eine Schrumpftemperatur von etwa 100°C und einen Schrumpfgrad in radialer Richtung von etwa 50 % besitzt.

5. Strahlerhalter nach einem der Ansprüche 1 bis 4, dadurch gekennzeichnet, daß der Schrumpfschlauch (5) mit einer Spitze (6) versehen ist.

6. Strahlerhalter nach Anspruch 5, dadurch gekennzeichnet, daß die Spitze (6) mit dem Schrumpfschlauch (5) verklebt ist oder daß ein hinterer Endteil (9) der Spitze (6) in den Schrumpfschlauch (5) ragt und der Schrumpfschlauch (5) auf diesen Endteil (9) aufgeschrumpft ist.

7. Strahlerhalter nach einem der Ansprüche 1 bis 6, dadurch gekennzeichnet, daß der Schrumpfschlauch (5) an einem Endteil mit einem Kupplungsteil (11) angeschlossen ist, der an dem Schrumpfschlauch (5) angeklebt ist oder mit einem Fortsatz (10) in den Schrumpfschlauch (5) ragt, wobei der Schlauch (5) auf den Fortsatz (10) aufgeschrumpft ist.

8. Strahlerhalter nach einem der Ansprüche 1 bis 7, dadurch gekennzeichnet, daß die Seeds (7) und die Füllteile (7') hintereinander ohne Abstand im Schrumpfschlauch (5) angeordnet sind.

9. Strahlerhalter nach einem der Ansprüche 1 bis 8, dadurch gekennzeichnet, daß die radiale Schrumpfbarkeit des Schrumpfschlauches (5) größer ist als seine Schrumpfbarkeit in Längsrichtung.

10. Strahlerhalter nach einem der Ansprüche 1 bis 9, dadurch gekennzeichnet, daß die Seeds (7) und/oder die Distanzstücke (7') mit Kennungen Markierungen, z.B. Farbmarkierungen, magnetischen Markierungen, vorzugsweise im Barcode, gekennzeichnet sind.

11. Verfahren zur Herstellung radioaktiver Strahlerhalter zum Einsatz in interstitiell eingebrachte Bestrahlungsnadeln nach einem der Ansprüche 1 bis 10, dadurch gekennzeichnet, daß zumindest ein radioaktives Seed (7), vorzugsweise eine Anzahl von radioaktiven Seeds (Strahlerelementen), in einen, vorzugsweise transparenten und gegebenenfalls bereits abgelängten Schrumpfschlauch (5) eingeführt werden und daß daraufhin der Schlauch auf das (die) Seed(s) unter dessen Lagefixierung thermisch aufgeschrumpft wird.

12. Verfahren nach Anspruch 11, dadurch gekennzeichnet, daß zusätzlich zu den Seeds (7) eine Anzahl von nicht radioaktiven Füllteilen bzw. Distanzstücken (7') in vorgegebener Aufeinanderfolge mit den Seeds in den Schrumpfschlauch eingebracht wird.

13. Verfahren nach Anspruch 11 oder 12, dadurch gekennzeichnet, daß vorzugsweise vor dem Einführen der Seeds -eine Spitze (6) und vorzugsweise nach dem Einführen der Seeds ein Kupplungsteil (11), insbesondere inclusive Codierung, mit dem abgelängten Schlauch (5), z.B. durch Kleben, verbunden oder in den abgelängten Schlauch eingesteckt und beim Schrumpfvorgang am Schlauch befestigt werden.

14. Verfahren nach einem der Ansprüche 11 bis 13, dadurch gekennzeichnet, daß vor dem Einbringen der Seeds (7) bzw. der Füllteile bzw. Distanzstücke (7') eine Seele, z.B. ein Stahldraht, in den Schrumpfschlauch (5) eingeführt wird, deren Durchmesser größer als der Durchmesser der Seeds und Füllteile bzw. Distanzstücke ist, und daß der Schrumpfschlauch auf den Durchmesser der Seele vorgeschrumpft wird.

15. Verfahren nach einem der Ansprüche 11 bis 14, dadurch gekennzeichnet, daß die Aktivität der Seeds (7) insbesondere unmittelbar vor dem Einbringen in den Schrumpfschlauch (5) gemessen wird und nur Seeds mit vorbestimmten Aktivitätswerten (z.B. im Bereich von ± 3% bezogen auf den gewünschten Wert) in den Schrumpfschlauch eingeführt werden.

16. Einrichtung zum Herstellen von Strahlerhaltern nach einem der Ansprüche 1 bis 10 zum Einsatz in interstitiell eingebrachte Bestrahlungsnadeln bzw. zur Durchführung des Verfahrens nach einem der Ansprüche 11 bis 15, dadurch gekennzeichnet, daß eine Führungshülse (4) vorgesehen ist, an bzw. in die ein Schrumpfschlauch (5) ansetzbar bzw. einsteckbar ist, und daß die Führungshülse (4) zur Einführung der Seeds (7) in den Schrumpfschlauch (5) an das Ende einer von einer Rütteleinrichtung (1) für radioaktive Seeds (7) kommenden, vorzugsweise durchsichtigen Transportleitung (13) anlegbar bzw. ankoppelbar ist und daß der Einrichtung eine Heizeinrichtung, z.B. ein Heißlufterzeuger, zum Schrumpfen des Schrumpfschlauches nachgeordnet ist, durch die der gefüllte Schlauch (5) mit einer Transporteinrichtung durchbewegbar ist.

17. Einrichtung nach Anspruch 16, dadurch gekennzeichnet, daß die Führungsteile (4) mittels einer Umschalteinrichtung bzw. einer Weiche (14) in willkürlicher Weise an die Transportleitung (13) der Rütteleinrichtungen (1) für die Seeds (7) und eine Transporteinrichtung (13') einer Rütteleinrichtung (12) für nichtaktive Füllteile bzw. Distanzstücke (7') anschließbar ist.

18. Einrichtung nach Anspruch 16 oder 17, dadurch gekennzeichnet, daß in der Transportleitung (13) bzw. der Transportleitung (13') knapp vor der Führungshülse (4) eine von einer die Transportleitungen (13,13') überwachenden Lichtschranke (8) betätigbare Sperrvorrichtung (2) vorgesehen ist.

## Claims

1. A seedholder for use in interstitially inserted radiation needles, comprising a plastics material sleeve (5) and at least one preferably elongated radioactive seed (7) disposed therein, characterized in that the plastics material sleeve is formed by a plastics material sheath or plastics material shrink tube (5) thermally shrunk onto the at least one seed (7) underneath its positioning means.

2. The seedholder according to claim 1, wherein a plurality of shrunk-in seeds (7) is consecutively disposed in the shrink tube (5).

3. The seedholder according to claim 1 or 2, wherein radioactive seeds (7) and non-radioactive fillers or spacers (7') preferably of the same diameter as the seeds (7) are disposed in the shrink tube (5) in predetermined succession.

4. The seedholder according to any one of the claims 1 to 3, wherein the preferably transparent shrink tube (5) has a shrinking temperature of about 100°C and a shrinkage degree in radial direction of about 50 percent.

5. The seedholder according to any one of the claims 1 to 4, wherein the shrink tube (5) is provided with a point (6).

6. The seedholder according to claim 5, wherein the point (6) is glued to the shrink tube (5) or a rear end portion (9) of the point (6) projects into the shrink tube (5) and the shrink tube (5) is shrunk onto said end portion (9).

7. The seedholder according to any one of the claims 1 to 6, wherein one end portion of the shrink tube (5) is connected to a coupling part (11) which is glued to the shrink tube (5) or projects into the shrink tube (5) with an extension (10), the tube (5) being shrunk onto the extension (10).

8. The seedholder according to any one of the claims 1 to 7, wherein the seeds (7) and the fillers (7') are disposed in the shrink tube (5) consecutively abutting one another.

9. The seedholder according to any one of the claims 1 to 8, wherein the radial shrinking capacity of the shrink tube (5) is greater than its shrinking capacity in longitudinal direction.

10. The seedholder according to any one of the claims 1 to 9, wherein the seeds (7) and/or the spacers (7') are characterized by means of iddentifications or markings, for instance color markings, magnetic markings, preferably in the bar code.

11. A process for the manufacture of radioactive seedholders for use in interstitially inserted radiation needles according to any one of the claims 1 to 10, characterized in that at least one radioactive seed (7), preferably a plurality of radioactive seeds (seed elements), is (are) inserted into a preferably transparent and optionally already cut-off shrink tube (5) and the tube is thereupon shrunk onto the seed(s) underneath its positioning means.

12. The process according to claim 11, wherein in addition to the seeds (7), a plurality of non-radioactive fillers or spacers (7') is inserted into the shrink tube together with the seeds in predetermined succession.

13. The process according to claim 11 or 12, wherein preferably prior to inserting the seeds, a point (6) and preferably after insertion of the seeds a coupling part (11), in particular including coding, is connected to the cut-off tube (5), for instance by means of gluing, or inserted into the cut-off tube and attached to the tube during the shrinking process.

14. The process according to any one of the claims 11 to 13, wherein prior to insertion of the seeds (7) or fillers or spacers (7'), a core, for instance a steel wire whose diameter is greater than the diameter of the seeds or fillers or spacers is inserted into the shrink tube (5) and the shrink tube is then preshrunk to the diameter of the core.

15. The process according to any one of the claims 11 to 14, wherein the activity of the seeds (7) is measured in particular immediately prior to insertion into the shrink tube (5) and only seeds with predetermined activity values ( for instance in the range of ± 3 percent based on the desired value) are introduced into the shrink tube.

16. A device for the manufacture of seedholders according to any one of the claims 1 to 10 for use in interstitially inserted radiation needles and for carrying out the process according to any one of the claims 11 to 15, characterized in that a guiding sleeve (4) onto or into which the shrink tube (5) is insertable is provided and that the guiding sleeve (4) is attachable or connectable to the end of a preferably transparent conveying line (13) leading from a vibrating means (1) for radioactive seeds (7) and a heating means, for instance a hot air generator for shrinking the shrink tube through which the filled tube (5) is convey able by a conveying means is arranged downstream from said means.

17. The device according to claim 16, wherein the guiding sleeve (4) is randomly connectable to the conveying line ((13) of the vibrating means (1) for the seeds (7) and a conveying means (13') of a vibrating means (12) for non-radioactive fillers or spacers (7') by means of a switching means or switch (14).

18. The device according to claim 16 or 17, wherein a blocking device (2) actuable by a light barrier (8) monitoring the conveying lines (13, 13') is provided in the conveying line (13) and the conveying line (13') immediately upstream from the guiding sleeve (4).

## Revendications

1. Magasin à sources radioactives à mettre en place dans des aiguilles d'irradiation introduites dans les tissus, comprenant une enveloppe de matière synthétique (5) et au minimum une source radioactive (7), avantageusement de forme allongée, disposée dans ledit magasin caractérisé en ce que l'enveloppe de matière synthétique est constituée par une gaine de matière synthétique rétractable (5) adaptée par rétraction sous l'effet de la chaleur sur la(les)dite(s) source(s) radioactive(s) (7) en la (les) fixant dans une position déterminée.

2. Magasin à sources radioactives selon la revendication 1, caractérisé en ce qu'un certain nombre de sources radioactives (7) sont disposées les unes à la suite des autres dans la gaine (5) qui leur adhère par rétraction.

3. Magasin à sources radioactives selon la revendication 1 ou 2, caractérisé en ce que des sources radioactives (7) et des éléments intercalaires ou éléments de remplissage non radioactifs (7'), avantageusement de même diamètre que les sources radioactives (7), sont disposés dans la gaine rétractable (5) dans un ordre donné.

4. Magasin à sources radioactives se,on une des revendications 1 à 3, caractérisé en ce que la gaine rétractable (5) avantageusement transparente présente une température de rétraction de 100°C environ et un coefficient de rétraction radiale de 50% environ.

5. Magasin à sources radioactives selon une des revendications 1 à 4, caractérisé en ce que la gaine rétractable (5) est munie d'un embout (6).

6. Magasin à sources radioactives selon la revendication 5, caractérisé en ce que l'embout (6) est collé a la gaine rétractable (5) ou que l'embout (6) a une queue (9) pénétrant dans la gaine rétractable (5) et que la gaine rétractable (5) est soudée par rétraction à ladite queue (9).

7. Magasin à sources radioactives selon une des revendications 1 à 6, caractérisé en ce que la gaine rétractable (5) est fixée par une de ses extrémités à un accouplement (11) collé à la gaine rétractable (5) ou ayant un appendice (10) pénétrant dans la gaine rétractable (5), la gaine (5) étant soudée par rétraction sur cet appendice (10).

8. Magasin à sources radioactives selon une des revendications 1 à 7, caractérisé en ce que les sources radioactives (7) et les éléments de remplissage (7') sont disposés les uns à la suite des autres en se touchant dans la gaine rétractable (5).

9. Magasin à sources radioactives selon une des revendications 1 à 8, caractérisé en ce que la rétractabilité de la gaine (5) est plus importante dans le sens radial que dans celui de la longueur.

10. Magasin à sources radioactives selon une des revendications 1 à 9, caractérisé en ce que les sources radioactives (7) et/ou les éléments intercalaires (7') portent des repères, des marques, p.ex. marques de couleur, marques magnétiques, avantageusement sous forme de codes barres.

11. Procédé pour la fabrication de magasins à sources radioactives à mettre en place dans des aiguilles d'irradiation introduites dans les tissus selon une des revendications 1 à 10, caractérisé en ce que l'on introduit au moins une source radioactive (7), avantageusement un certain nombre de sources radioactives (éléments irradiants) dans une gaine rétractable (5) avantageusement transparente, le cas échéant préalablement coupée à une longueur donnée, et que la gaine est ensuite adaptée par rétraction thermique sur la (les) source(s) radioactive(s), en la (les) fixant dans une position donnée.

12. Procédé selon la revendication 11, caractérisé en ce que, outre les sources radioactives (7), un certain nombre d'éléments intercalaires ou éléments de remplissage non radioactifs (7') sont introduits dans la gaine rétractable, dans un ordre déterminé par rapport aux sources radioactives.

13. Procédé selon la revendication 11 ou 12, caractérisé en ce que l'on fixe, avantageusement avant l'introduction des sources radioactives, un embout (6) et, avantageusement après l'introduction des sources radioactives, un accouplement (11), en particulier avec codage, à la gaine (5) coupée à une longueur déterminée, à laquelle ils sont assujettis, p.ex. collés, ou dans laquelle ils sont introduits et à laquelle ils sont fixés par rétraction de ladite gaine.

14. Procédé selon une des revendications 11 à 13, caractérisé en ce qu'une âme, p.ex. un fil d'acier dont le diamétre est supérieur à celui des sources radioactives et des éléments de remplissage ou éléments intercalaires est introduite dans la gaine rétractable (5) avant l'introduction des sources radioactives (7) ou des éléments de remplissage ou éléments intercalaires (7'), et que la gaine rétractable est rétrécie au préalable au diamètre de l'âme.

15. Procédé selon une des revendications 11 à 14, caractérisé en ce que l'activité des sources radioactives (7) est mesurée en particulier immédiatement avant leur introduction dans la gaine rétractable (5) et que seules des sources radioactives répondant aux valeurs voulues (p.ex. dont la radioactivité ne diffère pas de ±3% de la valeur demandée) sont introduites dans la gaine rétractable.

16. Dispositif pour la fabrication de magasins à sources radioactives selon une des revendications 1 à 10, à mettre en place dans des aiguilles d'irradiation introduites dans les tissus ou pour la réalisation du procédé selon une des revendications 11 à 15, caractérisé en ce qu'il est prévu une douille de guidage (4) sur laquelle une gaine rétractable (5) peut être montée ou dans laquelle elle peut être introduite, et que, pour permettre l'introduction des sources radioactives (7) dans la gaine rétractable (5), la douille de guidage (4) peut être adaptée ou accouplée à l'extrémité d'un conduit de transport (13) provenant d'un dispositif à secousses (1) pour sources radioactives (7), qui sera de préférence transparent, et qu'un système de chauffage, p.ex. une source d'air chaud, fait suite audit dispositif pour obtenir la rétraction de la gaine rétractable, un dispositif de transport assurant le déplacement de la gaine remplie (5) dans ledit système de chauffage.

17. Dispositif selon la revendication 16, caractérisé en ce que les éléments de guidage (4) peuvent être raccordés à volonté, au moyen d'un dispositif de commutation ou d'un aiguillage (14), au conduit de transport (13) du dispositif à secousses (1) d'alimentation en sources radioactives (7) et au conduit de transport (13') d'un dispositif à secousses (12) d'alimentation en éléments de remplissage ou éléments intercalaires non radioactifs (7').

18. Dispositif selon la revendication 16 ou 17, caractérisé en ce qu'un dispositif d'arrêt (2) commandé par une barrière photo-électrique (8) assurant la surveillance des conduits de transport (13, 13') est prévu dans le conduit de transport (13) et le conduit de transport (13') juste en avant de la douille de guidage (4).
